# EUROPÄISCHE PATENTANMELDUNG

(11) **EP 0 543 115 A2**
(43) Veröffentlichungstag der Anmeldung: **26.05.1993**
(21) Anmeldenummer: 92116549.4
(22) Anmeldetag: 25.09.1992
(51) Int. Cl.: G02B 27/10, G02B 27/14

(54) **Optisches System zur Kopplung von Laserstrahlquellen**

(30) Priorität: 25.09.1991 DE 4131968
(71) Anmelder: G. RODENSTOCK INSTRUMENTE GMBH, D-85521 Ottobrunn (DE)
(72) Erfinder: Reis, Werner, W-8000 München 50 (DE)
(74) Vertreter: Münich, Wilhelm, Dr.

(57) **Zusammenfassung**

Beschrieben wird ein optisches System mit wenigstens zwei Laserstrahlquellen, die Laserstrahlen mit einem nicht rotationssymmetrischen Querschnitt und unterschiedlicher Wellenlänge emittieren, die wenigstens ein wellenlängenselektives Koppelelement koaxial vereinigt.

Die Erfindung zeichnet sich dadurch aus, daß die Achsen der nicht rotationssymmetrischen Laserstrahlen der Laserstrahlquellen derart gegeneinander verdreht sind, daß das Lichtbündel nach dem oder den Koppelelementen ein zumindest annähernd rotationssymmetrische Form hat.

## Beschreibung

Die Erfindung bezieht sich auf ein optisches System mit wenigstens zwei Laserstrahlquellen, die Laserstrahlen mit einem nicht rotationssymmetrischen Querschnitt und unterschiedlicher Wellenlänge emittieren, die wenigstens ein wellenlängenselektives Koppelelement koaxial vereinigt.

Derartige Laserstrahlquellen sind beispielsweise Laserdioden, aber auch bestimmte Excimer-Laser.

Laserdioden - die auch als Halbleiterlaser bezeichnet werden - haben den Vorteil, daß sie vergleichsweise kostengünstig und unproblematisch im "Handling" sind. Nachteilig ist jedoch, daß zum gegenwärtigen Zeitpunkt noch keine Laserdioden mit einer Leistung, wie sie beispielsweise für ophthalmologische Behandlungen erforderlich ist, kostengünstig zur Verfügung stehen.

Deshalb ist vorgeschlagen worden, mehrere Laserdioden zu verwenden, deren Laserstrahlen wenigstens ein Koppelelement zu einem (mehr oder weniger) koaxialen Strahl vereinigt.

Aus der Beschreibungseinleitung der DE 33 00 581 C2 ist es in diesem Zusammenhang bekannt, hierzu Zwei Laserdioden zu verwenden, die Laserlicht mit unterschiedlicher Wellenlänge emittieren. Die von diesen Laserstrahlquellen ausgehenden Lichtbündel werden über einen dichroitischen Spiegel zusammengefaßt, der das Laserlicht der einen Wellenlänge hindurchtreten läßt und das Laserlicht der anderen Wellenlänge koaxial zu dem hindurchtretenden Laserstrahl umlenkt.

Keine Beachtung wird bei dem aus dieser Druckschrift bekannten Stand der Technik der Tatsache gewidmet, daß die von Laserdioden ausgehenden Lichtbündel einen stark von der Rotationssymmetrie abweichenden Querschnitt haben:
Die Laseraustrittsfläche entspricht in etwa der p-n-Übergangsschicht der Laserdiode. Dabei ist die Ausdehnung der Laseraustrittsfläche senkrecht zur p-n Übergangsschicht häufig sogar deutlich kleiner als die Laserwellenlänge (600 bis 1000 nm). Aufgrund von Beugungseffekten ist daher der Strahl sehr divergent; der Divergenzwinkel kann Werte bis zu 40° erreichen.

Andererseits weist die aktive Zone parallel zur p-n-Übergangsschicht wesentlich größere Abmessungen als senkrecht zur Übergangsschicht auf; beispielsweise betragen die Abmessungen parallel zur Übergangsschicht bei einer Laserdiode mit Leistung von 1 Watt ca 200 µm. Deshalb hat der aus einer Laserdiode austretende Laserstrahl nicht nur einen stark elliptischen Strahlquerschnitt, sondern weist auch unterschiedliche Divergenzwinkel auf; somit ergibt sich eine elliptische bis spaltförmige Intensitätsverteilung.

Darüberhinaus weist der Strahl eines Diodenlasers Astigmatismus auf, d.h. der Laserstrahl scheint für die Richtungen parallel und senkrecht zur p-n Übergangsschicht von zwei verschiedenen Punkten auszugehen. Um den Laserstrahl auf einen Fleck mit sehr kleinem Durchmesser fokussieren zu können, ist es erforderlich, diesen Astigmatismus zu korrigieren.

Es ist Aufgabe der Erfindung, ein optisches System mit wenigstens zwei Laserstrahlquellen, die Laserstrahlen mit einem nicht rotationssymmetrischen Querschnitt und unterschiedlicher Wellenlänge emittieren, die wenigstens ein wellenlängenselektives Koppelelement koaxial vereinigt, derart weiterzubilden, daß das zusammengefaßte, koaxiale Lichtbündel eine möglichst homogene Energieverteilung und eine für die Fokussierung günstige Strahlgeometrie aufweist.

Eine erfindungsgemäße Lösung dieser Aufgabe ist im Anspruch 1 angegeben. Weiterbildungen der Erfindung sind Gegenstand der Ansprüche 2 folgende.

Erfindungsgemäß sind die Achsen der nicht rotationssymmetrischen Laserstrahlen der Laserstrahlquellen, die beispielsweise gemäß Anspruch 2 Laserdioden sein können, derart gegeneinander verdreht, daß das Lichtbündel nach dem oder den Koppelelementen eine zumindest annähernd rotationssymmetrische Form hat.

Wie bereits ausgeführt, hat eine Laserdiode mit einer Leistung von 1 Watt eine Abstrahlcharakteristik, die zu einem Bündel mit einem Längen/Breiten-Verhältnis von 200:1 führt. Derart extrem nicht rotationssymmetrische Bündel lassen sich durch anamorphotische Abbildungen, wie bespielsweise den Einsatz von gekreuzten Zylinderlinsen nur schwer in eine wenigstens annähernd runde Form überführen; in der Regel ist auch beim Einsatz von Zylinderlinsen ein minimales Verhältnis Länge zu Breite von 20:1 kaum zu unterschreiten.

Erfindungsgemäß ist erkannt worden, daß es - um eine möglichst runde verlustarme Laserspotabbildung zu erhalten - sinnvoll ist, nicht nur die Gesamtleistung (Spaltlängen) auf mehrere Dioden zu verteilen, sondern auch, die Strahlen der einzelnen Laser gegeneinander verdreht in einen Strahlengang bzw. eine Lichtleitfaser einzukoppeln.

Dieser erfindungsgemäße Grundgedanke wird dadurch realisiert, daß eine geeignete Anordnung der Laserstrahlquellen, vorzugsweise in Form von Laserdioden, im optischen System zur Kopplung von Lichtquellen erfolgt. Dabei werden die Laseraustrittsöffnungen bzw. die Strahlquerschnitte der einzelnen Laserdioden so gedreht, daß eine annähernd symmetrische Geometrie bzw. eine annähernd homogene Intensitätsverteilung des Gesamtstrahls ergibt; dies wird beispielsweise beim Einsatz von drei Dioden durch eine Verdrehung der einzelnen Dioden bzw. deren p-n-Übergänge um jeweils 60° erreicht.

Aus optischen Abbildungsgründen (sphärische Aberation usw.) ist es günstig, die Dioden so auszuwählen, daß die Wellenlängen so nah wie möglich beieinander liegen, daß ihre Lichtstrahlen aber durch entsprechende wellenlängenselektive Teilerschichten sicher noch von einander getrennt werden können.

Hierzu ist es bevorzugt, wenn sich die Wellenlängen der einzelnen Laserstrahlquellen um ca. 10 bis 20 nm unterscheiden. Weiterhin ist es von Vorteil, wenn das oder die Koppelelemente dichroitische Spiegel sind, deren Transmissions/Reflexionskanten sich entsprechend, d.h. um ca. 10 bis 20 nm unterscheiden.

Hauptvorteile bei der Anwendung der Erfindung sind die homogenere Energieverteilung und die damit bessere Ausleuchtung im Laserspot, so daß man einen verzerrungsfreien Strahlquerschnitt erhält. Dies ist insbesondere in der Ophthalmologie bei der Koagualtion des Augenhintergrunds von Vorteil, d.h. "Überhitzungen" bestimmter Stellen auf dem Augenhintergrund vermieden werden.

Weiterhin können kostengünstige Einzeldioden verwendet werden; dies ist insbesondere deshalb vorteilhaft, da der Preis für leistungsstärkere Dioden derzeit progressiv nach oben tendiert, so daß die Kosten für mehrere vergleichsweise leistungsschwache Dioden, die entsprechende Zahl von Koppelelementen und die Justierung geringer sind als die Kosten einer gleich leistungsstarken Einzeldiode. Dies gilt umso mehr, wenn man den höheren Aufwand für die "Strahlformung" einer Einzeldiode in Ansatz bringt.

Ein zusätzliches Plus ist die Möglichkeit, die Dioden seriell oder parallel je nach Leistungsbedarf anzusteuern (mit den dadurch gegebenen Überlastungsreserven).

Darüberhinaus ist es durch die erfindungsgemäße Ausbildung häufig möglich, auf teure anamorphotische Systeme zu verzichten. Trotzdem ist eine problemlose Fokussierung des "vereinigten Bündels" in eine Lichtleitfaser möglich.

Die Erfindung wird nachstehend anhand eines Ausführungsbeispiels unter Bezugnahme auf die Zeichnung näher beschrieben, in der zeigen:
- Fig. 1: eine Prinzipdarstellung des zusammengefaßten Laserstrahls an der Austrittsöffnung des optischen Systems beim Einsatz von drei Laserstrahlquellen,
- Fig. 2: eine Ausführungsvariante eines optischen Systems zur Kopplung von drei Laserdioden
Zur Einstellung des in Fig. 1 gezeigten zusammengefaßten Strahlenbündels (5) werden drei um jeweils 60° zueinander verdrehte Laserdioden mit den zugehörigen Strahlquerschnitten (4) eingesetzt. Dies ist im einzelnen in Fig. 2 dargestellt:

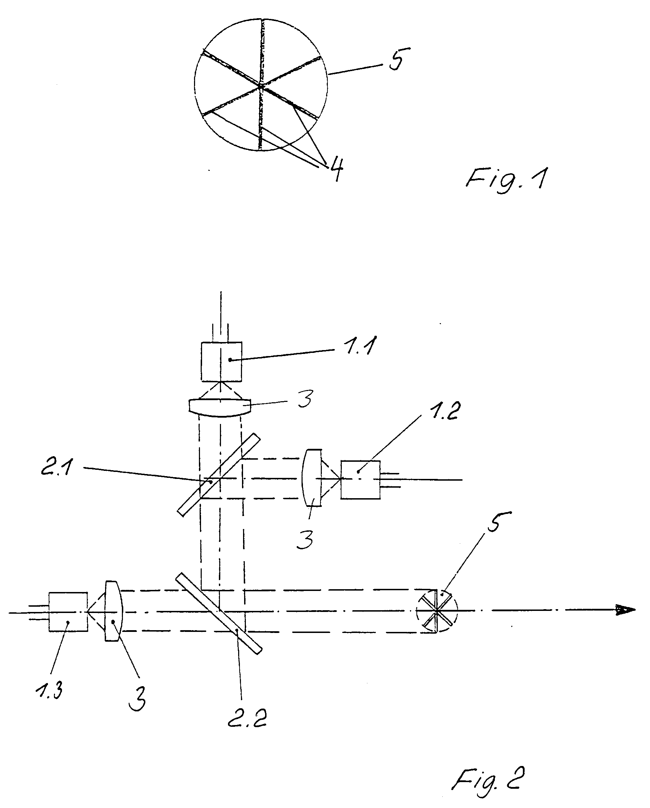

Die Ausführungsvariante eines optischen Systems zur Kopplung von Laserstrahlquellen nach Fig. 2 besteht im wesentlichen aus drei um jeweils 60° gedrehten Laserdioden 1.1 bis 1.3, zwei Teilerspiegeln 2.1 u. 2.2 und der jeweils zugehörigen Kollimatoroptik 3.

Unter Drehung der Laserdioden ist insbesondere zu verstehen, daß die p/n-Übergänge der Laserdioden (1) entsprechend gegeneinander verdreht sind.

Der Teilerspiegel 2.1 ist so beschichtet, daß er das Licht der Laserdiode 1.1 durchläßt und das Licht der Diode 1.2 reflektiert. Der Teilerspiegel 2.2 ist ähnlich aufgebaut, er läßt das Licht von 1.3 vollständig durch und reflektiert das Licht der Laserdioden 1.1 und 1.2, so daß sich ein koaxiales Bündel ergibt.

Beispielsweise können Laserdioden mit folgenden Wellenlängen verwendet werden:

| | |
|---|---|
| Diode 1.1 | = 770 nm |
| Diode 1.2 | = 810 nm |
| Diode 1.3 | = 850 nm |

Entsprechend gelten für die Teilerspiegel:
- Spiegel 2.1:: Transmission bis 790 nm
Reflexion ab 810 nm
- Spiegel 2.2:: Transmission bis 830 nm
Reflexion ab 840 nm
Vorstehend ist die Erfindung anhand eines Ausführungsbeispiels beschrieben worden; innerhalb des erfindungsgemäßen Grundgedankens sind selbstverständlich die verschiedensten Varianten möglich:

So ist es immer dann, wenn zwar eine homogene Energieverteilung des Strahls, aber keine annähernd beugungsbegrenzte Abbildung bzw. Fokussierung des Strahl erforderlich ist, auch möglich, nicht nur eine einzige, in den Figuren nicht dargestelle Fokussieroptik, sondern auch anstelle der jeder Laserdiode zugeordneten Kollimatoroptik eine einzige Kollimatoroptik zu verwenden.

Weiterhin können selbstverständlich auch Systeme mit zwei oder vier und mehr Laserdioden verwendet werden; beispielsweise bei vier Laserdioden werden dann die "langen" Achsen der einzelnen Laserstrahlen bevorzugt, aber nicht notwendigerweise um 90° gegeneinander verdreht.

## Patentansprüche

**1.** Optisches System mit wenigstens zwei Laserstrahlquellen, die Laserstrahlen mit einem nicht rotationssymmetrischen Querschnitt und unterschiedlicher Wellenlänge emittieren, die wenigstens ein wellenlängenselektives Koppelelement koaxial vereinigt,
dadurch **gekennzeichnet**, daß die Achsen der nicht rotationssymmetrischen Laserstrahlen (4) der Laserstrahlquellen (1) derart gegeneinander verdreht sind, daß das Lichtbündel nach dem oder den Koppelelementen ein zumindest annähernd rotationssymmetrische Form hat.

**2.** Optisches System nach Anspruch 1,
dadurch **gekennzeichnet**, daß die Laserstrahlquellen Laserdioden (1) sind.

**3.** Optisches System nach Anspruch 2,
dadurch **gekennzeichnet**, daß die p/n-Übergänge der Laserdioden (1) entsprechend gegeneinander verdreht sind.

**3.** Optisches System nach einem der Ansprüche 1 bis 3,
dadurch gekennzeichnet, daß beim Einsatz von drei Laserstrahlquellen (1.1 bis 1.3) die Achsen der einzelnen Laserstrahlen um jeweils 60° verdreht sind.

**5.** Optisches System nach einem der Ansprüche 1 bis 4,
dadurch **gekennzeichnet**, daß sich die Wellenlängen der einzelnen Laserstrahlquellen um ca 10 bis 20 nm unterscheiden.

**6.** Optisches System nach Anspruch 5,
dadurch **gekennzeichnet**, daß das oder die Koppelelemente dichroitische Spiegel sind, deren Transmissions/Reflexionskanten sich um ca. 10 bis 20 nm unterscheiden.

**7.** Optisches System nach einem der Ansprüche 1 bis 6,
dadurch **gekennzeichnet**, daß das vereinigte Lichtbündel (5) in eine Lichtleitfaser fokussiert ist.
